# EUROPÄISCHE PATENTSCHRIFT

(11) **EP 2 724 660 B1**
(45) Veröffentlichungstag und Bekanntmachung des Hinweises auf die Patenterteilung: **17.06.2015**
(21) Anmeldenummer: 13189008.9
(22) Anmeldetag: 16.10.2013
(51) Int. Cl.: A61B 1/005, A61B 1/00

(54) **Endoskopisches Instrument**
Endoscopic instrument
Instrument endoscopique

(30) Priorität: 26.10.2012 DE 102012110255
(43) Veröffentlichungstag der Anmeldung: 30.04.2014
(73) Patentinhaber: Karl Storz GmbH & Co. KG, 78532 Tuttlingen (DE)
(72) Erfinder: Friedrich, Christina, 72184 Eutingen (DE); Schwarz, Peter, 78532 Tuttlingen-Nendingen (DE)
(74) Vertreter: Witte, Weller & Partner Patentanwälte mbB

(56) Entgegenhaltungen:
- EP-A1- 2 039 284
- EP-A2- 0 078 017
- US-A- 4 503 842

## Beschreibung

Die vorliegende Erfindung betrifft ein endoskopisches Instrument mit einem Bedienstück mit einem Bedienelement, ferner mit einem Instrumentenschaft mit einem betätigbaren Abschnitt, wobei der Instrumentenschaft mit dem Bedienstück verbunden ist, einem Stellelement, einem Zugelement, das mit dem Stellelement und dem Abschnitt des Instrumentenschafts mechanisch gekoppelt ist, so dass eine Verlagerung des Stellelements eine Betätigung des Abschnitts mittels einer Kraftübertragung über das Zugelement bewirken kann, und außerdem mit einem Aktuator, der mit dem Stellelement gekoppelt ist, so dass eine Betätigung des Aktuators eine Verlagerung des Stellelements durch Kraftübertragung vom Aktuator auf das Stellelement bewirken kann.

Ein solches Endoskop ist aus US 4,499,895 bekannt.

Endoskopische Instrumente mit flexiblem oder starrem Instrumentenschaft werden sowohl in der Industrie als auch im medizinischen Bereich verwendet. Beispielsweise werden flexible Endoskope im veterinärmedizinischen Bereich bei der gastroendoskopischen Untersuchung von Großtieren eingesetzt. Diese Endoskope besitzen häufig an einem distalen Ende ihres Instrumentenschafts einen distalen Endabschnitt, der in einem Endstück endet. Das Endstück stellt den distalen Teil des Endoskops dar, der in den zu untersuchenden Körper eingeführt wird. Es weist üblicherweise das distale Ende einer Endoskopoptik sowie zum Teil auch Absaug-, Spül- und Werkzeugkanäle auf.

Um eine größtmögliche Flexibilität bezüglich einer räumlichen Ausrichtung des Endstücks bei der Untersuchung gewährleisten zu können, ist der Endabschnitt des Instrumentenschafts üblicherweise ablenkbar ausgebildet. Durch ein Ablenken, bzw. allgemein Betätigen, des Abschnitts zu dem restlichen Instrumentenschaft, insbesondere durch ein Abwinkeln oder Abkrümmen, kann ein Teil des Instrumentenschafts, insbesondere das Endstück, nach Wunsch ausgerichtet werden. Bei diesem Ablenken ist eine vorsichtige Vorgehensweise eines Anwenders notwendig, damit kein Gewebe verletzt wird, das sich um den Endabschnitt herum befindet. Es ist daher wichtig, dass das Betätigen des Abschnitts sehr präzise steuerbar ist.

Die Ablenkung des Abschnitts erfolgt bei flexiblen und starren Endoskopen gemäß dem Stand der Technik über ein Zugelement, insbesondere über Bowdenzüge. Das Zugelement ist mit einem Stellelement, insbesondere einem Lenkgetriebe, verbunden. Das Zugelement ist dabei häufig an einem Schnurrad befestigt. Durch eine Betätigung des Bedienelements am Bedienstück des Endoskops wird das Stellelement bewegt. Durch eine rotatorische Bewegung des Stellelements ergibt sich eine translatorische Bewegung des Zugelements. Die Bewegung des Zugelements hat wiederum eine Ablenkung des Abschnitts zur Folge.

Die eingangs genannte US 4,499,895 zeigt ein elektrisches Endoskop, dessen Bedienung über einen Hebel erfolgt, der mit dem Stellelement gekoppelt ist. Wenn der Hebel gegenüber dem Stellelement verlagert wird, wird eine Biegung des Hebels oder eine Widerstandsänderung eines Potentiometers erfasst und dadurch eine Unterstützung des Stellelements durch einen Aktuator eingestellt. Der gezeigte Aufbau ist allerdings sehr unhandlich und erfordert im Fehlerfall einen speziellen Lösemechanismus, um das Endoskop noch bedienen zu können.

Weitere ähnliche Endoskope sind aus Dokumenten US 4,503,842 A, EP 2 039 284 A1 und EP 0 078 017 A2 bekannt.

US 7,331,924 beschreibt ein elektrisches Endoskop mit einem ablenkbaren distalen Abschnitt. Die Bedienung des Endoskops erfolgt hier über einen Trackball, dessen Verlagerung durch den Daumen oder einen anderen Finger eines Benutzers von einer elektrischen Schaltung erfasst wird. In Abhängigkeit von der rotatorischen Verlagerung des Trackballs, wie sie vom Benutzer gewählt wird, wird in einer Ablenkungssteuerung die Ansteuerung eines Motors bestimmt, der eine Verlagerung des Zugelements und damit ein Ablenken des distalen Abschnitts bewirkt.

Der Nachteil eines solchen elektrischen Endoskops ist jedoch, dass die Bedienung als weniger intuitiv empfunden wird, da die Bedienung eines elektrischen Endoskops, z.B. beim Anstoß an Gewebe, nicht die Rückmeldung in Form einer Gegenkraft am Bedienelement liefert, die der Benutzer von mechanischen Endoskopen kennt. Dieselbe Problematik ergibt sich auch bei elektrischen Endoskopen, die über einen Joystick bedient werden. Dabei sei lediglich beispielsweise auf das Dokument US 6,932,761 hingewiesen. Elektrische Endoskope haben außerdem den Nachteil, dass sie sich bei einem Defekt möglicherweise nur schwer aus dem Hohlraum herausziehen lassen, wenn der distale Abschnitt abgelenkt ist.

Bei mechanischen Endoskopen erfolgt die Ablenkung des Abschnitts ausschließlich über eine mechanische Krafteinwirkung des Benutzers auf eine außen liegende Handhabe am Bedienstück des Endoskops. Dafür ist das Stellelement üblicherweise fest auf einer Welle der Handhabe angeordnet. Betätigt der Benutzer die Handhabe, so entsteht eine rotatorische Verlagerung des Stellelements und dadurch wiederum die translatorische Bewegung des Zugelements.

Rein mechanische Endoskope können aber je nach Länge des Endoskops und der Lage des Instrumentenschafts erhebliche Kräfte für eine Betätigung erfordern. Zudem erzeugt die mechanische Betätigung des Abschnitts automatisch eine gewisse Rückstellkraft in Richtung der nichtabgelenkten Position (Nullposition) des Endoskops. Ferner bildet der Abschnitt bei der Ablenkung mit dem Zugelement ein FederDämpfer-System, das Energie beim Spannen speichert und beim Entspannen freigibt. Dadurch können eine Anlauftotzeit oder ein Nachlaufweg beim Ablenken des Endabschnitts entstehen. Dies führt dazu, dass der Benutzer die Ablenkung des Abschnitts, die er eigentlich einstellen möchte, um eine bestimmte Stelle betrachten zu können, nur näherungsweise oder iterativ einstellen kann.

Es ist daher eine Aufgabe der vorliegenden Erfindung, ein endoskopisches Instrument der eingangs genannten Art dahingehend weiterzubilden, dass der Benutzer zwar eine Unterstützung, wie bei einer elektrischen Betätigung, bei der Bedienung erhält, dabei aber nicht auf eine mechanische Rückmeldung durch das Endoskop verzichten muss. Außerdem ist es eine Aufgabe, dass das Endoskop selbst bei einem Ausfall der elektrischen Steuerung weiterhin bedienbar bleibt.

Die Aufgabe wird gelöst durch ein eingangs genanntes endoskopisches Instrument mit einem Zwischenelement, das mit dem Bedienelement und dem Stellelement wirkverbunden ist, so dass eine vom Benutzer auf das Bedienelement ausgeübte Kraft über das Zwischenelement auf das Stellelement übertragen werden kann, um das Stellelement zu verlagern, wobei das Zwischenelement einen ersten Abschnitt und einen zweiten Abschnitt aufweist, der erste Abschnitt mit dem zweiten Abschnitt verbunden ist, der zweite Abschnitt einen größeren Biegewiderstand aufweist als der erste Abschnitt und der erste Abschnitt ein Messelement zum Messen einer Biegung des ersten Abschnitts aufweist, wobei das Stellelement eine erste Anschlagseinrichtung, die einen ersten Bewegungsbereich des ersten Abschnitts relativ zum Stellelement definiert, und eine zweite Anschlagseinrichtung aufweist, die einen zweiten Bewegungsbereich des zweiten Abschnitts relativ zum Stellelement definiert, und der erste Bewegungsbereich kleiner ist als der zweite Bewegungsbereich.

Die Erfindung bietet mehrere Besonderheiten, die insbesondere in der Ausgestaltung des Zwischenelements und des Stellelements und im Zusammenspiel von Bedienelement, Zwischenelement und Stellelement gesehen werden. Um dies zu verdeutlichen, werden nachfolgend die verschiedenen Betätigungsmöglichkeiten des endoskopischen Instruments erläutert.

Als erstes soll die Möglichkeit eines rein motorischen Betriebs betrachtet werden. Dabei betätigt ein Benutzer das Bedienelement derart, dass sich das Zwischenelement geringfügig gegenüber dem Stellelement verlagert, insbesondere rotatorisch verlagert. Wenn der erste Abschnitt von der ersten Anschlagseinrichtung bei seiner Verlagerung gebremst oder gestoppt wird, stellt sich eine zumindest geringfügige Durchbiegung des ersten Abschnitts ein, da der Benutzer weiterhin eine Kraft auf das Bedienelement ausübt.

Es sei an dieser Stelle darauf hingewiesen, dass im Rahmen dieser Anmeldung der Begriff Biegung insbesondere eine Dehnung und eine Stauchung einschließt. Außerdem soll der Begriff "Kraft" auch ein Drehmoment umfassen, da sich dieses insbesondere aus der Länge eines Hebelarms multipliziert mit einer Kraft ergibt.

Die Biegung des ersten Abschnitts kann von dem Messelement erfasst werden. Dabei ist es vorteilhaft, wenn nicht nur das alleinige Vorhandensein einer Biegung ermittelt wird, sondern auch die Richtung der Durchbiegung, insbesondere ob es sich um eine Dehnung oder Stauchung handelt. Besonders bevorzugt ist es, wenn auch die Stärke der Biegung ermittelt wird, da dies einen Rückschluss auf die vom Benutzer aufgebrachte Kraft ermöglicht.

Aus der Kenntnis der Biegung kann auf die vom Benutzer durchgeführte Betätigung geschlossen werden. Dabei kann insbesondere die vom Benutzer gewählte Betätigungsrichtung und/oder die vom Benutzer aufgebrachte Kraft erkannt werden. Da somit die vom Benutzer gewünschte Betätigung des endoskopischen Instruments erkannt wird, wird dann der Aktuator derart angesteuert, dass er die vom Benutzer gewünschte Verlagerung des Stellelements vornimmt. Der Benutzer bedient das endoskopische Instrument dann rein motorisch.

Bei einer weiteren Betriebsmöglichkeit wird das Stellelement durch eine Kombination von motorischer Betätigung und durch die Kraft des Benutzers bewirkt. Dabei gilt zunächst wieder, dass eine Biegung des ersten Abschnitts zu einer motorischen Unterstützung führt. Nun kann es allerdings sein, dass der Benutzer eine so große Kraft aufbringt, dass nicht nur der erste Abschnitt in Kontakt mit der ersten Anschlagseinrichtung steht, sondern zusätzlich der zweite Abschnitt mit der zweiten Anschlagseinrichtung in Kontakt tritt. Da der zweite Abschnitt gegenüber dem ersten Abschnitt einen größeren Biegewiderstand aufweist, überträgt sich zumindest ein Teil der vom Benutzer aufgebrachten Kraft vom Bedienelement über das Zwischenelement auf das Stellelement. Dies bedeutet, dass die Verlagerung des Stellelements sowohl durch den Aktuator als auch durch zumindest einen Teil der vom Benutzer aufgebrachten Kraft erfolgt. Außerdem kann so verhindert werden, dass die Kraft des Benutzers zu einer plastischen Verformung des ersten Abschnitts führt, die den ersten Abschnitt beschädigt. Der zweite Abschnitt stellt also auch einen Überlastschutz für den ersten Abschnitt dar.

Wie bereits ausgeführt wurde, weist der zweite Abschnitt einen größeren Biegewiderstand auf als der erste Abschnitt. Dabei ist es bevorzugt, wenn der zweite Abschnitt einen deutlich größeren Biegewiderstand aufweist als der erste Abschnitt. Es ist vorteilhaft, wenn der Biegewiderstand des zweiten Abschnitts mindestens das 1,5-fache, bevorzugt mindestens das Doppelte, besonders bevorzugt mindestens das Dreifache und insbesondere mindestens das Fünffache des Biegewiderstands des ersten Abschnitts beträgt. Der Biegewiderstand soll bevorzugt dahingehend verstanden werden, dass er einen Zusammenhang zwischen einer aufgebrachten Kraft und einer daraus resultierenden Verformung beschreibt, wobei die Verformung bei gleicher Kraft und höherem Biegewiderstand kleiner ist. Beispielhaft bedeutet dies, dass bei zwei Quadern von gleicher Länge, aber mit unterschiedlich großen quadratischen Querschnitten, der Quader mit dem größeren Querschnitt einen höheren Biegewiderstand aufweist. Ein unterschiedlicher Biegewiderstand kann bevorzugt auch durch die Wahl unterschiedlicher Materialien für den ersten und den zweiten Abschnitt erzielt werden, dies sowohl bei gleichen oder unterschiedlichen Geometrien von erstem und zweitem Abschnitt.

Schließlich soll der Fall einer Betätigung betrachtet werden, die ausschließlich durch die Kraft des Benutzers erfolgt, also rein manuell. Dabei wird angenommen, dass die motorische Unterstützung durch den Aktuator entfällt. Der Grund hierfür kann ein Ausfall des Aktuators oder seiner Ansteuerung sein oder, wie es bei einer bevorzugten Ausführungsform möglich ist, weil der Benutzer die motorische Unterstützung abgeschaltet hat. Bei dieser Betriebsmöglichkeit findet weiterhin eine Biegung des ersten Abschnitts statt, jedoch keine Verlagerung des Stellelements durch den Aktuator. Daher ist es erforderlich, dass der zweite Abschnitt mit der zweiten Anschlagseinrichtung in Kontakt tritt, um die Kraft des Benutzers zumindest zum größten Teil auf das Stellelement zu übertragen.

Wie anhand der Ausführungsbeispiele noch verdeutlicht wird, wird es als vorteilhaft angesehen, wenn die jeweilige Anschlagseinrichtung durch zwei Vorsprünge auf dem Stellelement realisiert ist und sich der jeweilige Abschnitt zwischen diesen zwei Vorsprüngen bewegt oder an diesen anliegt. Bei einer anderen vorteilhaften Ausgestaltung ist zumindest eine der Anschlagseinrichtungen als Ausnehmung oder Nut in dem Stellelement ausgebildet und der jeweilige Abschnitt greift mit einem Vorsprung in diese Ausnehmung bzw. diese Nut ein. Bei einer weiteren vorteilhaften Ausgestaltung ist zumindest eine Anschlagseinrichtung als ein Vorsprung auf dem Stellelement angeordnet und ist das Ende des jeweiligen Abschnitts in der Art einer Gabel mit zwei Zinken ausgebildet, wobei der Vorsprung zwischen dem Zinken angeordnet ist. Schließlich ist bei einer weiteren vorteilhaften Ausgestaltung die Anschlagseinrichtung als ein Vorsprung auf dem Stellelement angeordnet, der in eine Ausnehmung oder Nut des jeweiligen Abschnitts eingreift.

Es sei darauf hingewiesen, dass das Stellelement mehrere Bestandteile aufweisen kann, insbesondere Verbindungselemente und Kraftübertragungselemente wie zum Beispiel Achsen, Scheiben oder Zahnräder. Bei einer bevorzugten Ausführungsform weist das Stellelement ein Schnurrad auf. Bei einer weiteren bevorzugten Ausführungsform weist das Stellelement zudem ein Getriebe auf, das eine vom Zwischenelement auf das Stelleelement übertragene Kraft auf das Schnurrad überträgt.

Schließlich sei darauf hingewiesen, dass es vorteilhaft ist, wenn das Material des ersten Abschnitts so gewählt wird, dass die Stärke der Biegung proportional zu einer aufgebrachten Kraft ist. Außerdem sei darauf hingewiesen, dass es bei bestimmten Ausführungsformen ausreichend ist, wenn der erste Bewegungsbereich nur geringfügig kleiner ist als der zweite Bewegungsbereich, da lediglich verhindert werden soll, dass die Bewegung des Stellelements direkt über den zweiten Abschnitt erfolgt, ohne dass eine Biegung des ersten Abschnitts auftritt.

Damit ist die Aufgabe vollständig gelöst.

Bei einer bevorzugten Ausgestaltung sind der erste Abschnitt und der zweite Abschnitt einstückig ausgebildet, insbesondere aus genau einem Werkstoff ausgebildet.

Dies ermöglicht eine kostengünstige Fertigung und erleichtert die Montage. Die Biegewiderstände des ersten und des zweiten Abschnitts lassen sich durch die entsprechende Wahl des Materials und die Dimensionen des jeweiligen Abschnitts, insbesondere Länge, Höhe und Breite, einstellen und bei Bedarf anpassen. Insbesondere kann durch eine geeignete Wahl der Dimensionen und/oder des Werkstoffes sichergestellt werden, dass ausschließlich eine Verformung im elastischen Bereich erfolgt und die Abschnitte nicht dauerhaft verformt bzw. beschädigt werden.

Bei einer weiteren vorteilhaften Ausgestaltung der Erfindung hat der erste Abschnitt ständig mechanischen Kontakt zu der ersten Anschlagseinrichtung.

Diese Ausgestaltung ist vorteilhaft, da sich so bereits sehr geringe Auslenkungen des Bedienelements erfassen lassen.

Bei einer weiteren vorteilhaften Ausgestaltung ist das Messelement auf dem ersten Abschnitt angeordnet, und der elektrische Widerstand des Messelements ändert sich bei Änderung einer Form des Messelements.

Wenngleich es bei einer bevorzugten Ausgestaltung vorgesehen ist, das Messelement innerhalb des ersten Abschnitts anzuordnen, wird es als besonders vorteilhaft angesehen, wenn das Messelement auf dem ersten Abschnitt angeordnet ist. Dies ermöglicht es insbesondere, das Zwischenelement im Hinblick auf die gewünschten Biegewiderstände und/oder geometrische Erwägungen auszugestalten und dann ein bevorzugtes Messelement auf dem ersten Abschnitt anzuordnen. Das Messelement wird dabei bevorzugt an einer Seite des ersten Abschnitts angebracht, an der sich eine Biegung besonders gut detektieren lässt, also insbesondere an Flächen, die bei der Biegung gedehnt oder gestaucht werden. Hierfür eignen sich insbesondere solche Flächen, die zumindest in etwa senkrecht zu einer Verlagerungsrichtung des Zwischenelements stehen. Das Messelement wird vorzugsweise als Dehnungsmessstreifen ausgebildet, der auf den ersten Abschnitt aufgebracht ist. Dabei kann der erste Abschnitt auch als Biegebalken verstanden werden.

Bei einer bevorzugten Ausgestaltung weist das Messelement ein erstes Messelementteil und ein zweites Messelementteil auf, die derart an zwei verschiedenen Seiten des ersten Abschnitts angeordnet sind, das sich bei einer Biegung des ersten Abschnitts eine Änderung einer Form des ersten Messelementteils von einer Änderung einer Form des zweiten Messelementteils unterscheidet.

Bei dieser Ausgestaltung kann eine Biegung besonders zuverlässig und genau ermittelt werden. Zudem können aufgrund der zwei Messelementteile Störungen und Umwelteinflüsse, zum Beispiel durch Änderung der Umgebungstemperatur kompensiert werden. In diesem Zusammenhang ist es vorteilhaft, wenn eine Differenz eines ersten Messwerts des ersten Messelementteils und eines zweiten Messwerts des zweiten Messelementteils gebildet wird. Es ist besonders vorteilhaft, wenn die beiden Messelementteile an gegenüberliegenden Seiten des ersten Abschnitts angeordnet sind, wobei die Seiten bevorzugt zumindest in etwa senkrecht zu einer Verlagerungsrichtung des Zwischenelements stehen.

Bei einer weiteren bevorzugten Ausgestaltung ist die zweite Anschlagseinrichtung einstellbar, um den zweiten Bewegungsbereich des zweiten Abschnitts relativ zum Stellelement zu variieren.

Auf diese Weise kann individuell eingestellt werden, wie weit eine Biegung des ersten Abschnitts möglich sein soll, bevor eine zumindest teilweise Kraftübertragung über den zweiten Abschnitt auf das Stellelement erfolgt. Bei einigen Ausgestaltungen ist es vorteilhaft, dem zweiten Abschnitt einen größeren Spielraum einzuräumen, da dies eine ausgeprägtere Biegung des ersten Abschnitts ermöglicht und daher eine rein motorische Steuerung in einem breiten Bereich möglich ist. Bei anderen Ausgestaltungen ist es vorteilhaft, den zweiten Bewegungsbereich gering zu halten, insbesondere dann, wenn man langfristig die Präzision der Biegungsmessung sicherstellen möchte. Es kann so verhindert werden, dass sich der erste Abschnitt zu sehr biegt und dadurch die Präzision der Biegungsmessung leidet. Die Anschlagseinrichtung wird bevorzugt eingestellt, indem Anschlagselemente verwendet werden, bei denen die Position einer Anschlagsfläche veränderbar ist, insbesondere durch Drehen, Schrauben oder Schieben.

Bei einer weiteren Ausgestaltung weist das Stellelement ein Getriebe auf, insbesondere ein Getriebe mit einem positiven Übersetzungsverhältnis.

Diese Ausgestaltung ermöglicht eine besonders gute Anpassung, wie die vom Benutzer aufgebrachte Kraft auf das Zugelement übertragen wird. Dabei ist es besonders vorteilhaft, dass das Getriebe als Kombination von Außenzahnrad und Innenzahnrad realisiert ist. Dabei sei an dieser Stelle darauf hingewiesen, dass bei einer anderen bevorzugten Ausführungsform der Aktuator ein Getriebe aufweist.

Bei einer weiteren vorteilhaften Ausgestaltung fallen eine erste Rotationsachse des Stellelements und eine zweite Rotationsachse des Aktuators zusammen.

Diese Ausgestaltung ermöglicht einen kostengünstigen Aufbau und eine einfache Kraftübertragung des Aktuators auf das Stellelement. Insbesondere fallen dabei eine fünfte Rotationsachse eines Schnurrads und die zweite Rotationsachse des Aktuators zusammen

Bei einer weiteren vorteilhaften Ausgestaltung der Erfindung weist das Stellelement ein zumindest in etwa kreisförmiges Trägerelement auf, auf dem die erste Anschlagseinrichtung und die zweite Anschlagseinrichtung zumindest in etwa in Umfangsrichtung des Trägerelements angeordnet sind.

Diese Ausgestaltung ermöglicht eine kostengünstige Fertigung des endoskopischen Instruments und einen zuverlässigen Betrieb. Dabei ist es bei einigen Ausführungsformen bevorzugt, wenn das Trägerelement in direktem mechanischen Kontakt mit dem Zugelement steht, und insbesondere das Trägerelement als Schnurrad ausgeführt ist. Bei anderen Ausgestaltungen ist es vorteilhaft, insbesondere bei Verwendung eines Getriebes, dass das Trägerelement nicht in direktem mechanischem Kontakt mit dem Zugelement steht, insbesondere das Trägerelement ein Bauteil separat vom Schnurrad ist.

Bei einer weiteren vorteilhaften Ausgestaltung der Erfindung ist eine Steuereinrichtung dafür ausgebildet, einen Messwert einer Biegung des ersten Abschnitts zu erfassen und den Aktuator unter Berücksichtigung des Messwerts anzusteuern.

Diese Ausgestaltung ermöglicht eine vorteilhafte Realisierung des rein motorischen bzw. des kombinierten Betriebs. Die Steuereinrichtung wertet im Zusammenspiel mit dem Messelement einen Messwert oder ein Messsignal aus, das eine Biegung des ersten Abschnitts anzeigt. Dabei wird zumindest erkannt, dass der Benutzer das endoskopische Instrument betätigen möchte. Es ist bevorzugt, dabei ebenfalls die Richtung der gewünschten Betätigung zu ermitteln und insbesondere auch eine Information über die vom Benutzer aufgebrachte Kraft zu ermitteln. Die Steuereinrichtung erhält demnach eine Information darüber, dass der Benutzer das endoskopische Instrument betätigt und bevorzugt wie er es betätigt. Die Steuereinrichtung ist daher dafür ausgebildet, den Aktuator so anzusteuern, dass die vom Benutzer gewünschte Betätigung des endoskopischen Instruments motorisch vorgenommen oder zumindest unterstützt wird.

Bei einer weiteren vorteilhaften Ausgestaltung fallen eine dritte Rotationsachse des Bedienelements und eine vierte Rotationsachse des Zwischenelements zusammen.

Diese Ausgestaltung ermöglicht einen kostengünstigen Aufbau und eine einfache Kraftübertragung des Bedienelements auf das Zwischenelement.

Bei einer weiteren vorteilhaften Ausgestaltung weist das Messelement mindestens einen Dehnungsmessstreifen auf.

Diese Ausgestaltung ermöglicht eine einfache Erfassung einer Biegung des ersten Abschnitts. Dabei wird der Dehnungsmessstreifen bevorzugt mit einer Spannung beaufschlagt, wobei sich der resultierende Strom bei einer Biegung des ersten Abschnitts ändert, da der Dehnungsmessstreifen gedehnt (oder gestaucht) wird und dabei seinen elektrischen Widerstand verändert. Diese Ausgestaltung ist unter anderem deswegen vorteilhaft, weil die Messung mittels eines Dehnungsmessstreifens einfach und zuverlässig durchgeführt werden kann. Bei einigen Ausführungsformen ist es vorteilhaft, wenn sowohl das erste Messelementteil als auch das zweite Messelementteil jeweils als Dehnungsmessstreifen ausgebildet sind. Bei einer weiteren vorteilhaften Ausgestaltung wird mindestens ein Polymerleiter in den zu biegenden Abschnitt integriert und wird die Biegung über dessen Widerstandsänderung erfasst.

Bei einer weiteren vorteilhaften Ausgestaltung sind der erste Abschnitt und der zweite Abschnitt zumindest in etwa balkenförmig ausgebildet, laufen auf einen gemeinsamen Punkt zu und stehen bezogen auf diesen gemeinsamen Punkt in einem Winkel zueinander.

Diese Ausgestaltung ist einfach zu realisieren, insbesondere im Hinblick auf eine vorteilhafte direkte Verbindung von erstem und zweitem Abschnitt. Dabei ist es bevorzugt, wenn sich der erste und der zweite Abschnitt tatsächlich an dem gemeinsamen Punkt berühren, doch können auch andere Ausgestaltungen vorteilhaft sein, die eine Verbindung von erstem und zweitem Abschnitt zum Beispiel über eine Brücke oder einen gemeinsamen Halter realisieren.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung näher dargestellt und werden in der nachfolgenden Beschreibung näher erläutert. Es zeigen:
- Fig. 1: ein Ausführungsbeispiel eines flexiblen Endoskops mit einem ablenkbaren distalen Abschnitt,
- Fig. 2: das Endoskop gemäß Fig. 1 in vereinfachter schematischer teilweiser Schnittdarstellung,
- Fig. 3: eine erste Ausführungsform von Zwischenelement und Stellelement,
- Fig. 4: eine zweite Ausführungsform von Zwischenelement und Stellelement im Ruhezustand,
- Fig. 5: die zweite Ausführungsform im ausgelenkten Zustand,
- Fig. 6: eine dritte Ausführungsform von Zwischenelement und Stellelement,
- Fig. 7: eine vierte Ausführungsform von Zwischenelement und Stellelement,
- Fig. 8: eine fünfte Ausführungsform von Zwischenelement und Stellelement,
- Fig. 9: eine erste Ausführungsform einer Anordnung von Bedienelement, Zwischenelement, Stellelement und Aktuator in der Seitenansicht,
- Fig. 10: eine vereinfachte Darstellung des Getriebes gemäß der sechsten Ausführungsform, und
- Fig. 11: eine zweite Ausführungsform einer Anordnung von Bedienelement, Zwischenelement, Stellelement und Aktuator in der Seitenansicht.

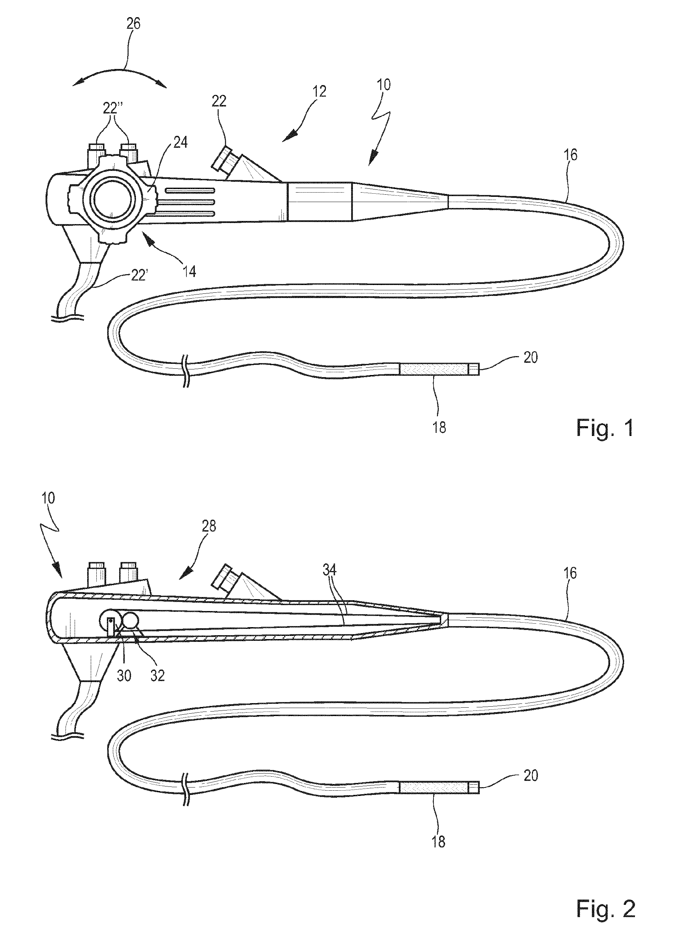

Fig. 1 zeigt ein endoskopisches Instrument 10 mit einem Bedienstück 12, mit einem Bedienelement 14 und einem flexiblen Instrumentenschaft 16. Es sei darauf hingewiesen, dass die Erläuterungen im Rahmen der Erfindung in gleicher Weise für einen starren Instrumentenschaft (nicht gezeigt) zutreffen.

Das endoskopische Instrument 10 wird zu Untersuchungs- und/oder Operationszwecken in medizinischen Verfahren verwendet. In dem Instrumentenschaft 16 verlaufen eine nicht dargestellte Endoskopoptik in Form von Lichtleitfasern, Bildleitern, verschiedene Kanäle, wie ein Saug- und ein Spülkanal, und ein Instrumentenkanal. Der Instrumentenschaft 16 ist proximal mit dem Bedienstück 12 verbunden und erstreckt sich distal bis in einen ablenkbaren Abschnitt 18, bei dem es sich hier insbesondere um einen Endabschnitt handelt.

Dieser weist ein Endstück 20 in Form einer Abschlussbuchse auf. Das Endstück 20 ist der Bereich des Instrumentenschafts 16, in dem die Lichtleitfasern, die Bildleiter und Kanäle enden. Der Instrumentenschaft 16 ist in den Figuren nur teilweise dargestellt. Das Bedienstück 12 weist einen Anschluss 22, ein Versorgungskabel 22' und Tasten 22" auf. Der Anschluss 22 führt zu einem Instrumentenkanal. Durch ihn können Instrumente in den Instrumentenschaft 16 und bis durch das Endstück 20 hindurch geführt werden. Dadurch können im Bereich vor dem Endstück 20 beispielsweise Operationen durchgeführt werden.

Das Versorgungskabel 22' beinhaltet verschiedene Arten von Versorgungen, wie beispielsweise eine elektrische Versorgung, Lichtleiter, Saug- und Spülschläuche und/oder Datenleitungen. Die Bildgebung des endoskopischen Instruments 10 erfolgt über einen nicht dargestellten Bildsensor im Inneren des Bedienstücks 12 oder im Endstück 20, wobei die Bilddaten über das Versorgungskabel 22' nach außen geleitet werden, insbesondere zu einer nicht dargestellten Kamerasteuerungseinheit (camera control unit, CCU).

Für ein Steuern des Ablenkens des Abschnitts 18 ist das Bedienelement 14 vorgesehen, wobei hier eine Ausführungsform mit einer Handhabe 24 zu sehen ist, die in die Richtungen des Doppelpfeils 26 gedreht werden kann. Durch Drehen des Bedienelements 14 wird der ablenkbare Abschnitt 18 auf/ab oder links/rechts abgelenkt. Die jeweilige Ablenkung korrespondiert hier mit der Drehrichtung des Bedienelements 14, entweder gegen oder im Uhrzeigersinn. Bei einer bevorzugten Ausführungsform wird zusätzlich zum gezeigten Bedienelement 14 ein nicht gezeigtes, weiteres Bedienelement verwendet, so dass über ein weiteres Stellelement und ein weiteres Zugelement Bewegungen auf/ab und links/rechts kombiniert werden können.

Wie in Fig. 2 stark vereinfacht dargestellt ist, sind in einem proximalen Endbereich 28 des Bedienstücks 12 ein Stellelement 30 und ein Aktuator 32 angeordnet. Das Stellelement 30 ist mit einem Zugelement 34 verbunden, das durch den Instrumentenschaft 16 hindurchgeführt ist und sich bis in den Endabschnitt 18 hinein erstreckt. Das Zugelement 34 ist um einen Teil des Stellelements 30 herum geführt und hier einstückig ausgebildet.

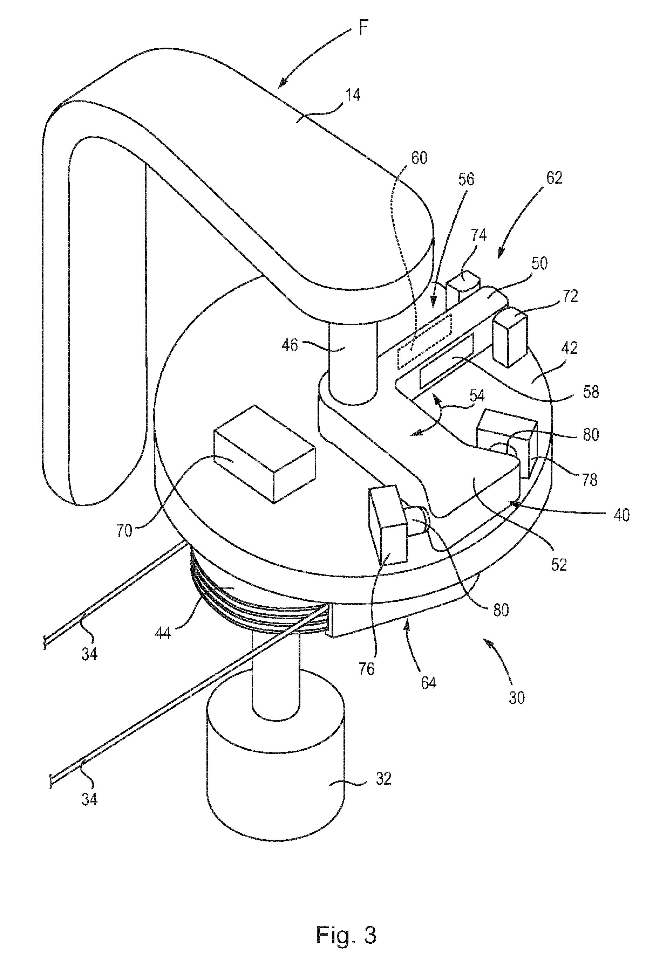

Fig. 3 zeigt ein erstes Ausführungsbeispiel eines Zwischenelements 40 und eines Stellelements 30. Von dem Stellelement 30 ist hier ein zumindest in etwa kreisförmiges Trägerelement 42 und ein Schnurrad 44 zu erkennen.

Das Zwischenelement 40 ist mit dem Bedienelement 14 über eine Bedienhebelwelle 46 fest verbunden. Das Bedienelement 14 ist zudem mit dem Stellelement 30 wirkverbunden, so dass eine vom Benutzer auf das Bedienelement 14 ausgeübte Kraft F über das Zwischenelement 40 auf das Stellelement 30 übertragen werden kann, um das Stellelement 30 zu verlagern, hier insbesondere das Schnurrad 44 zu verlagern.

Das Zwischenelement 40 weist einen ersten Abschnitt 50 und einen zweiten Abschnitt 52 auf. Der erste Abschnitt 50 ist mit dem zweiten Abschnitt 52 verbunden, hier einstückig aus einem gemeinsamen Werkstoff ausgebildet. Der zweite Abschnitt 52 weist - hier bezogen auf eine Bewegungsrichtung 54 des Zwischenelements 40 - einen größeren Biegewiderstand auf als der erste Abschnitt 50.

Der erste Abschnitt 50 weist ein Messelement 56 zum Messen einer Biegung des ersten Abschnitts 50 auf. Das Messelement 56 weist hier ein erstes Messelementteil 58 und ein zweites Messelementteil 60 auf. Da das zweite Messelementteil 60 in dieser Ansicht vom ersten Abschnitt 50 verdeckt ist, wird es lediglich gestrichelt dargestellt. Wie zu erkennen ist, ist das Messelement 56 auf dem ersten Abschnitt 50 angeordnet, und bei einer Änderung einer Form des Messelements 56 ändert sich der elektrische Widerstand des Messelements 56. Das Messelement 56, konkret das erste Messelementteil 58 und das zweite Messelementteil 60, ist jeweils bevorzugt als Dehnungsmessstreifen ausgebildet.

Es ist zu erkennen, dass das erste Messelement 58 und das zweite Messelement 60 an zwei verschiedenen Seiten des ersten Abschnitts 50 angeordnet sind. Dies führt dazu, dass sich bei einer Biegung des ersten Abschnitts 50 eine Änderung einer Form des ersten Messelements 58 von einer Änderung einer Form des zweiten Messelements 60 unterscheidet. Konkret bedeutet dies, dass eine Biegung des ersten Abschnitts 50 insbesondere entweder zu einer Dehnung des ersten Messelementteils 58 und zu einer Stauchung des zweiten Messelementteils 60 führt oder zu einer Stauchung des ersten Messelementteils 58 und einer Dehnung des zweiten Messelementteils 60.

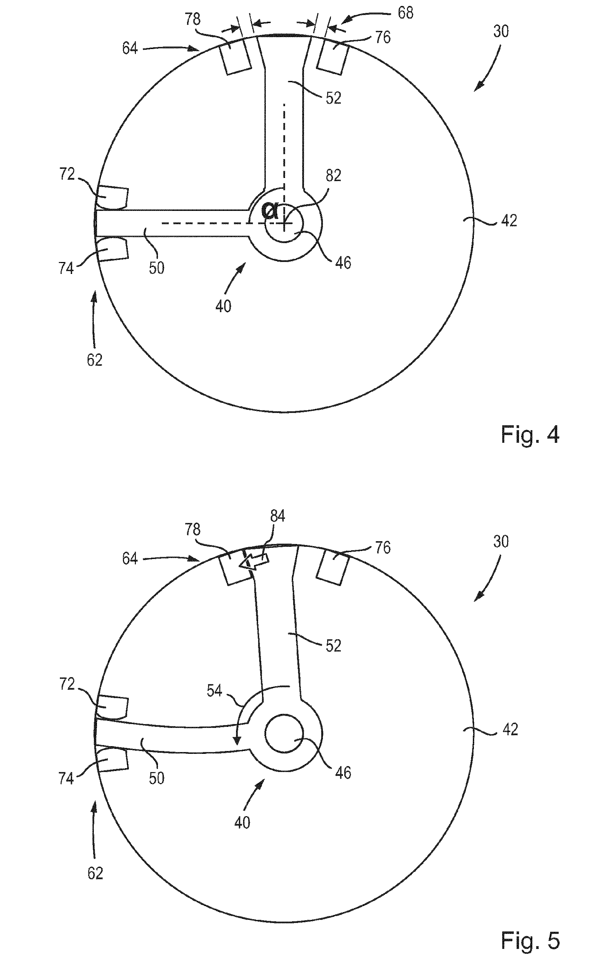

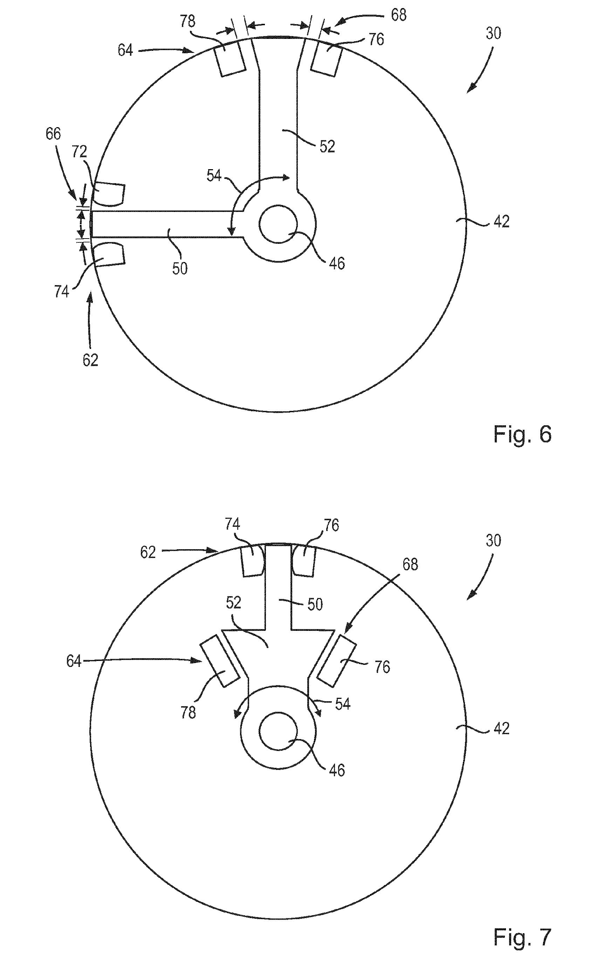

Das Stellelement 30 weist eine erste Anschlagseinrichtung 62 und eine zweite Anschlagseinrichtung 64 auf, die bei dieser Ausführungsform auf einem Trägerelement 42 angeordnet sind. Wie im Hinblick auf die Figuren 4 und 6 besonders gut zu erkennen ist, definiert die erste Anschlagseinrichtung 62 einen ersten Bewegungsbereich 66 des ersten Abschnitts 50 relativ zum Stellelement 30. Die zweite Anschlagseinrichtung 64 definiert einen zweiten Bewegungsbereich 68 des zweiten Abschnitts 52 relativ zum Stellelement 30. Wie nachfolgend noch gezeigt wird, ist der erste Bewegungsbereich 66 kleiner als der zweite Bewegungsbereich 68. Bei dieser Ausführungsform steht der erste Abschnitt 50 ständig in mechanischem Kontakt zu der ersten Anschlagseinrichtung 62, so dass der erste Bewegungsbereich 66 nahezu null oder null ist.

Das endoskopische Instrument 10 weist des Weiteren eine Steuereinrichtung 70 auf, die dafür ausgebildet ist, einen Messwert oder ein Messsignal einer Biegung des ersten Abschnitts 50 zu erfassen und den Aktuator 32, hier ein Elektromotor, unter Berücksichtigung des Messwerts bzw. des Messsignals anzusteuern. Die elektrischen Verbindungsleitungen zwischen der Steuereinrichtung 70 und dem Messelement 56 sind zum Zwecke einer besseren Übersichtlichkeit nicht dargestellt. Es ist bevorzugt, die elektrische Verbindung zumindest abschnittsweise mittels einer Platine bereitzustellen. Die Ansteuerung des Aktuators 32 wurde bereits ausführlich für die Betriebsmöglichkeiten rein motorisch, kombiniert und rein manuell erläutert und soll hier nicht wiederholt werden.

Die erste Anschlagseinrichtung 62 und die zweite Anschlagseinrichtung 64 sind hier in Umfangsrichtung des Trägerelements 42 angeordnet. Die erste Anschlagseinrichtung 62 weist einen ersten Mitnehmer 72 und einen zweiten Mitnehmer 74 auf, die hier als Vorsprünge ausgebildet sind und zwischen denen ein Bereich des ersten Abschnitts 50 zu liegen kommt. Die zweite Anschlagseinrichtung 64 ist einstellbar, um den zweiten Bewegungsbereich 68 des zweiten Abschnitts 52 relativ zum Stellelement 30 zu variieren. Dafür weist die zweite Anschlagseinrichtung 64 ein erstes Anschlagselement 76 und ein zweites Anschlagselement 78 auf, die jeweils über eine symbolisch dargestellte Madenschraube 80 einstellt werden können. Je weiter die Madenschrauben 80 aus dem jeweiligen Anschlagselement 76, 78 herausgedreht werden, desto kleiner wird der zweite Bewegungsbereich 68.

Fig. 4 zeigt eine zweite Ausführungsform von Zwischenelement 40 und Stellelement 30 in der Draufsicht, die in ihrer Funktion der ersten Ausführungsform gemäß Fig. 3 entspricht.

In der Draufsicht ist zu erkennen, dass die zumindest in etwa balkenförmigen Abschnitte 50, 52 auf einen gemeinsamen gedachten Punkt 82 zulaufen und bezogen auf diesen gemeinsamen Punkt 82 in einem Winkel α zueinander stehen. Der Winkel α beträgt hier ungefähr 90°. Wird das Zwischenelement 40 lediglich so weit gegenüber dem Stellelement 30 verlagert, dass der zweite Bewegungsbereich 68 nicht überschritten wird, findet lediglich eine Biegung des ersten Abschnitts 50 statt, wenn der Benutzer über das Bedienelement 14 eine Kraft F aufbringt. Die Biegung wird erkannt, und der Aktuator 32 wird zur motorischen Bewegung des Stellelements 30, hier insbesondere des Schnurrads 44 angesteuert.

Fig. 5 zeigt die zweite Ausführungsform gemäß Fig. 4, wenn die Kraft des Benutzers so groß ist, dass der kombinierte Betrieb eintritt. Es ist zu erkennen, dass der erste Abschnitt 50 nun gebogen ist und zumindest ein Teil der Kraft F, die der Benutzer über das Bedienelement 14 aufbringt, vom zweiten Abschnitt 52 auf das zweite Anschlagselement 78 der zweiten Anschlagseinrichtung 64 übertragen wird. Dies ist mittels des Pfeils 84 symbolisiert. Falls der Benutzer seine Kraft F weiter erhöht, wird diese Kraft im Wesentlichen über den zweiten Abschnitt 52 auf die zweite Anschlagseinrichtung 64 übertragen und führt nicht bzw. nur in einem geringen Ausmaß zu einer weiteren Biegung des ersten Abschnitts 50. Dies stellt einen Überlastschutz für den ersten Abschnitt 50 dar.

Fig. 6 zeigt eine dritte Ausführungsform, die im Wesentlichen der Ausführungsform gemäß Fig. 4 entspricht, wobei nun allerdings ein sichtbarer erster Bewegungsbereich 62 vorhanden ist. Wie bereits erläutert, soll darauf geachtet werden, dass der erste Bewegungsbereich 62 nicht größer als der zweite Bewegungsbereich 68 ist, da sonst die Situation eintreten könnte, dass der Benutzer nur den rein manuellen Betrieb zur Verfügung hat, da der erste Abschnitt 50 zu keinem Zeitpunkt eine Biegung erfährt.

Fig. 7 zeigt eine vierte Ausführungsform von Zwischenelement 40 und Stellelement 30. Es ist hier zu erkennen, dass der erste und der zweite Abschnitt 50, 52 auch hintereinander, also entlang einer geraden Strecke angeordnet sein können.

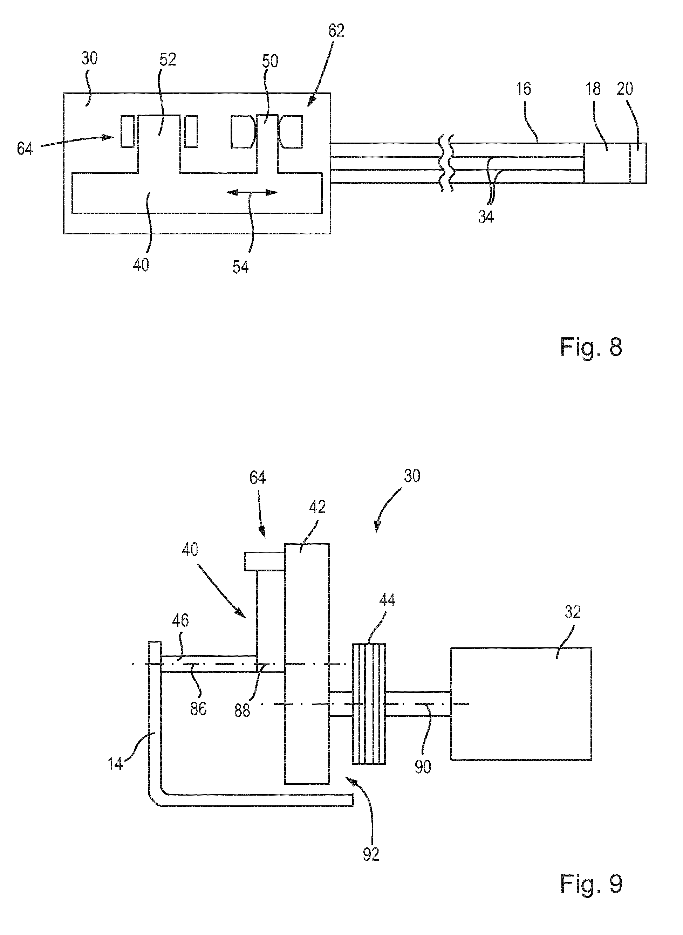

Fig. 8 zeigt eine fünfte Ausführungsform von Zwischenelement 40 und Stellelement 30. Dabei ist hier eine Möglichkeit gezeigt, die Erfindung auch für eine lineare Verlagerung zu nutzen. Der Aktuator 32, nicht gezeigt, kann auch hier als Elektromotor ausgeführt sein, insbesondere indem die angetriebene Welle des Elektromotors ein Zahnrad aufweist, das in eine Zahnstange am Stellelement 30 eingreift. Es könnte bevorzugt auch ein Linearmotor zum Einsatz kommen.

Fig. 9 zeigt eine erste Ausführungsform einer Anordnung von Bedienelement 14, Zwischenelement 40, Stellelement 30 und Aktuator 32 in der Seitenansicht. Dabei ist zu erkennen, dass eine Rotationsachse 86 des Bedienelements und eine Rotationsachse 88 des Zwischenelements 40 zusammenfallen. Außerdem ist eine Rotationsachse 90 des Aktuators eingezeichnet. Das Stellelement 30 weist hier ein Getriebe 92 auf. Wie in der nachfolgenden Figur noch verdeutlicht wird, handelt es sich um ein übersetztes Getriebe 92, d.h., eine Rotation des Bedienelements 14 um einen ersten Winkel führt zu einer größeren Rotation des Schnurrads 44.

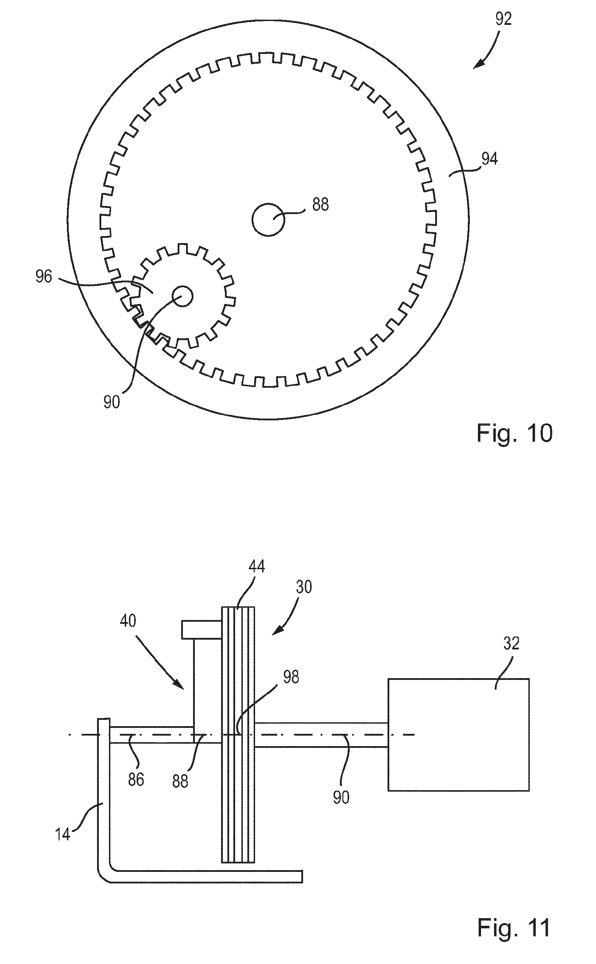

Fig. 10 zeigt eine Ausführungsform des Getriebes 92 gemäß Fig. 9. Dabei weist das Getriebe ein Innenzahnrad 94 und ein Zahnrad 96 auf. Durch eine entsprechende Wahl der Zahnräder 94, 96 kann eine gewünschte Übersetzung einfach realisiert werden.

Fig. 11 zeigt eine zweite Ausführungsform einer Anordnung von Bedienelement 14, Zwischenelement 40, Stellelement 30 und Aktuator 32 in der Seitenansicht. Bei dieser Ausgestaltung fallen die Rotationsachse 90 des Aktuators 32 und eine Rotationsachse 98 des Stellelements 30 zusammen. Ferner ist erkennen, dass die Rotationsachsen 90, 98 auch mit den Rotationsachsen 86, 88 zusammenfallen. Eine weitere Besonderheit ist hier, dass das Stellelement 30 als Schnurrad 44 realisiert ist.

Insgesamt wird ein endoskopisches Instrument 10 aufgezeigt, das einen rein motorischen, kombinierten und einen rein manuellen Betrieb ermöglicht. Zudem ist auch in einem Fehlerfall sichergestellt, dass der Benutzer das endoskopische Instrument 10 ohne Unterbrechung und ohne Einschränkung trotz fehlender Unterstützung durch den Aktuator 32 sicher bedienen kann.

## Patentansprüche

1. Endoskopisches Instrument (10) mit
einem Bedienstück (12) mit einem Bedienelement (14),
einem Instrumentenschaft (16) mit einem betätigbaren Abschnitt (18), wobei der Instrumentenschaft (16) mit dem Bedienstück (12) verbunden ist, einem Stellelement (30),
einem Zugelement (34), das mit dem Stellelement (30) und dem Abschnitt (18) des Instrumentenschafts (16) mechanisch gekoppelt ist, so dass eine Verlagerung des Stellelements (30) eine Betätigung des Abschnitts (18) mittels einer Kraftübertragung über das Zugelement (34) bewirken kann,
einem Aktuator (32), der mit dem Stellelement (30) gekoppelt ist, so dass eine Betätigung des Aktuators (32) eine Verlagerung des Stellelements (30) durch Kraftübertragung vom Aktuator (32) auf das Stellelement (30) bewirken kann, und
einem Zwischenelement (40), das mit dem Bedienelement (14) und dem Stellelement (30) wirkverbunden ist, so dass eine vom Benutzer auf das Bedienelement (14) ausgeübte Kraft (F) über das Zwischenelement (40) auf das Stellelement (30) übertragen werden kann, um das Stellelement (30) zu verlagern,
wobei das Zwischenelement (40) einen ersten Abschnitt (50) und einen zweiten Abschnitt (52) aufweist, der erste Abschnitt (50) mit dem zweiten Abschnitt (52) verbunden ist, und der erste Abschnitt (50) ein Messelement (56) zum Messen einer Biegung des ersten Abschnitts (50) aufweist,
wobei das Stellelement (30) eine erste Anschlagseinrichtung (62), die einen ersten Bewegungsbereich (66) des ersten Abschnitts (50) relativ zum Stellelement (30) definiert, und eine zweite Anschlagseinrichtung (64) aufweist, die einen zweiten Bewegungsbereich (68) des zweiten Abschnitts (50) relativ zum Stellelement (30) definiert,
**dadurch gekennzeichnet, dass**
der zweite Abschnitt (52) einen größeren Biegewiderstand aufweist als der erste Abschnitt (50), und dass
der erste Bewegungsbereich (66) kleiner ist als der zweite Bewegungsbereich (68).

2. Endoskopisches Instrument nach Anspruch 1, wobei der erste Abschnitt (50) und der zweite Abschnitt (52) einstückig ausgebildet sind, insbesondere aus genau einem Werkstoff ausgebildet sind.

3. Endoskopisches Instrument nach Anspruch 1 oder 2, wobei der erste Abschnitt (50) ständig mechanischen Kontakt zu der ersten Anschlagseinrichtung (62) hat.

4. Endoskopisches Instrument nach einem der vorhergehenden Ansprüche, wobei das Messelement (56) auf dem ersten Abschnitt (50) angeordnet ist und sich bei Änderung einer Form des Messelements (56) der elektrische Widerstand des Messelements (56) ändert.

5. Endoskopisches Instrument nach einem der vorhergehenden Ansprüche, wobei das Messelement (56) ein erstes Messelementteil (58) und ein zweites Messelementteil (60) aufweist, die derart an zwei verschiedenen Seiten des ersten Abschnitts (50) angeordnet sind, dass sich bei einer Biegung des ersten Abschnitts (50) eine Änderung einer Form des ersten Messelementteils (58) von einer Änderung einer Form des zweiten Messelementteils (60) unterscheidet.

6. Endoskopisches Instrument nach einem der vorhergehenden Ansprüche, wobei die zweite Anschlagseinrichtung (64) einstellbar ist, um den zweiten Bewegungsbereich (68) des zweiten Abschnitts (52) relativ zum Stellelement (30) zu variieren.

7. Endoskopisches Instrument nach einem der vorhergehenden Ansprüche, wobei das Stellelement (30) ein Getriebe (92) aufweist, insbesondere ein Getriebe (92) mit einem positiven Übersetzungsverhältnis.

8. Endoskopisches Instrument nach einem der vorhergehenden Ansprüche, wobei eine erste Rotationsachse (98) des Stellelements (30) und eine zweite Rotationsachse (90) des Aktuators (32) zusammenfallen.

9. Endoskopisches Instrument nach einem der vorhergehenden Ansprüche, wobei das Stellelement (30) ein zumindest in etwa kreisförmiges Trägerelement (42) aufweist, auf dem die erste Anschlagseinrichtung (62) und die zweite Anschlagseinrichtung (64) zumindest in etwa in Umfangsrichtung des Trägerelements (42) angeordnet sind.

10. Endoskopisches Instrument nach einem der vorhergehenden Ansprüche, ferner mit einer Steuereinrichtung, die dafür ausgebildet ist, einen Messwert einer Biegung des ersten Abschnitts (50) zu erfassen und den Aktuator (32) unter Berücksichtigung des Messwerts anzusteuern.

11. Endoskopisches Instrument nach einem der vorhergehenden Ansprüche, wobei eine dritte Rotationsachse (86) des Bedienelements (14) und einer vierten Rotationsachse (88) des Zwischenelements (40) zusammenfallen.

12. Endoskopisches Instrument nach einem der vorhergehenden Ansprüche, wobei das Messelement (56) mindestens einen Dehnungsmessstreifen aufweist.

13. Endoskopisches Instrument nach einem der vorhergehenden Ansprüche, wobei der erste Abschnitt (50) und der zweite Abschnitt (52) zumindest in etwa balkenförmig ausgebildet sind, auf einen gemeinsamen Punkt (82) zulaufen und bezogen auf diesen gemeinsamen Punkt (82) in einem Winkel (α) zueinander stehen.

## Claims

1. Endoscopic instrument (10) with
a control section (12) having a control element (14),
an instrument shaft (16) having an actuatable portion (18), wherein the instrument shaft (16) is connected to the control section (12),
an adjustment element (30)
a pull element (34), which is mechanically coupled to the adjustment element (30) and to the portion (18) of the instrument shaft (16), such that a movement of the adjustment element (30) can cause an actuation of the portion (18) via a force transmission via the pull element (34),
an actuator (32), which is coupled to the adjustment element (30), such that an actuation of the actuator (32) can cause a movement of the adjustment element (30) by force transmission from the actuator (32) to the adjustment element (30), and
an intermediate element (40), which is operatively connected to the control element (14) and to the adjustment element (30) such that a force (F) applied to the control element (14) by the user can be transmitted to the adjustment element (30) via the intermediate element (40) in order to move the adjustment element (30),
wherein the intermediate element (40) has a first portion (50) and a second portion (52), the first portion (50) is connected to the second portion (52), and the first portion (50) has a measurement element (56) for measuring a flexion of the first portion (50),
wherein the adjustment element (30) has a first abutment device (62), which defines a first movement range (66) of the first portion (50) relative to the adjustment element (30), and a second abutment device (64), which defines a second movement range (68) of the second portion (52) relative to the adjustment element (30),
**characterized by** the second portion (52) having a greater bending resistance than the first portion (50) and the first movement range (66) being smaller than the second movement range (68).

2. Endoscopic instrument of claim 1, wherein the first portion (50) and the second portion (52) are formed in one piece, in particular made from exactly one material.

3. Endoscopic instrument of claim 1 or 2, wherein the first portion (50) is continuously in mechanical contact with the first abutment device (62).

4. Endoscopic instrument of any preceding claim, wherein the measurement element (56) is arranged on the first portion (50) and wherein an electrical resistance of the measurement element (56) changes in the event of a change of shape of the measurement element (56).

5. Endoscopic instrument of any preceding claim, wherein the measurement element (56) has a first measurement element part (58) and a second measurement element part (60), which are arranged on two different sides of the first portion (50) in such a way that, in the event of a flexion of the first portion (50), a change of shape of the first measurement element part (58) differs from a change of shape of the second measurement element part (60).

6. Endoscopic instrument of any preceding claim, wherein the second abutment device (64) is adjustable in order to vary the second movement range (68) of the second portion (52) relative to the adjustment element (30).

7. Endoscopic instrument of any preceding claim, wherein the adjustment element (30) comprises a gear (92), in particular a gear (92) with a positive transmission ratio.

8. Endoscopic instrument of any preceding claim, wherein a first rotation axis (98) of the adjustment element (30) and a second rotation axis (90) of the actuator (32) coincide.

9. Endoscopic instrument of any preceding claim, wherein the adjustment element (30) has an at least approximately circular support element (42), on which the first abutment device (62) and the second abutment device (64) are arranged at least approximately in the circumferential direction of the support element (42).

10. Endoscopic instrument of any preceding claim, further with a controller configured to detect a measured value of a flexion of the first portion (50) and to trigger the actuator (32) on the basis of the measured value.

11. Endoscopic instrument of any preceding claim, wherein a third rotation axis (86) of the control element (14) and a fourth rotation axis (88) of the intermediate element (40) coincide.

12. Endoscopic instrument of any preceding claim, wherein the measurement element (56) has at least one strain gauge.

13. Endoscopic instrument of any preceding claim, wherein the first portion (50) and the second portion (52) are configured at least approximately in a beam shape, converge on a common point (82) and, in relation to this common point (82), are at an angle (α) to each other.

## Revendications

1. Instrument endoscopique (10), comprenant :
une pièce de commande (12) avec un élément de commande (14),
une tige d'instrument (16) avec une portion (18) pouvant être actionnée, la tige d'instrument (16) étant connectée à la pièce de commande (12),
un élément de réglage (30),
un élément de traction (34) qui est accouplé mécaniquement à l'élément de réglage (30) et à la portion (18) de la tige d'instrument (16), de telle sorte qu'un déplacement de l'élément de réglage (30) puisse provoquer un actionnement de la portion (18) au moyen d'un transfert de force par le biais de l'élément de traction (34),
un actionneur (32), qui est accouplé à l'élément de réglage (30) de telle sorte qu'un actionnement de l'actionneur (32) puisse provoquer un déplacement de l'élément de réglage (30) par transfert de force de l'actionneur (32) à l'élément de réglage (30), et
un élément intermédiaire (40) qui est en liaison fonctionnelle avec l'élément de commande (14) et l'élément de réglage (30), de telle sorte qu'une force (F) exercée par l'utilisateur sur l'élément de commande (14) puisse être transmise par le biais de l'élément intermédiaire (40) à l'élément de réglage (30) afin de déplacer l'élément de réglage (30),
l'élément intermédiaire (40) présentant une première portion (50) et une deuxième portion (52), la première portion (50) étant connectée à la deuxième portion (52), et la première portion (50) présentant un élément de mesure (56) pour mesurer une flexion de la première portion (50),
l'élément de réglage (30) présentant un premier dispositif de butée (62) qui définit une première plage de mouvement (66) de la première portion (50) par rapport à l'élément de réglage (30), et un deuxième dispositif de butée (64) qui définit une deuxième plage de mouvement (68) de la deuxième portion (50) par rapport à l'élément de réglage (30),
**caractérisé en ce que**
la deuxième portion (52) présente une plus grande résistance à la flexion que la première portion (50), et **en ce que**
la première plage de mouvement (66) est inférieure à la deuxième plage de mouvement (68).

2. Instrument endoscopique selon la revendication 1, dans lequel la première portion (50) et la deuxième portion (52) sont réalisées d'une seule pièce, en particulier à partir d'un matériau unique.

3. Instrument endoscopique selon la revendication 1 ou 2, dans lequel la première portion (50) présente un contact mécanique constant avec le premier dispositif de butée (62).

4. Instrument endoscopique selon l'une quelconque des revendications précédentes, dans lequel l'élément de mesure (56) est disposé sur la première portion (50) et dans le cas d'une variation d'une forme de l'élément de mesure (56), la résistance électrique de l'élément de mesure (56) varie.

5. Instrument endoscopique selon l'une quelconque des revendications précédentes, dans lequel l'élément de mesure (56) présente une première partie d'élément de mesure (58) et une deuxième partie d'élément de mesure (60), qui sont disposées sur deux côtés différents de la première portion (50) de telle sorte que dans le cas d'une flexion de la première portion (50), une variation d'une forme de la première partie d'élément de mesure (58) soit différente d'une variation d'une forme de la deuxième partie d'élément de mesure (60).

6. Instrument endoscopique selon l'une quelconque des revendications précédentes, dans lequel le deuxième dispositif de butée (64) peut être ajusté afin de faire varier la deuxième plage de mouvement (68) de la deuxième portion (52) par rapport à l'élément de réglage (30).

7. Instrument endoscopique selon l'une quelconque des revendications précédentes, dans lequel l'élément de réglage (30) présente un engrenage (92), notamment un engrenage (92) avec un rapport de démultiplication positif.

8. Instrument endoscopique selon l'une quelconque des revendications précédentes, dans lequel un premier axe de rotation (98) de l'élément de réglage (30) et un deuxième axe de rotation (90) de l'actionneur (32) coïncident.

9. Instrument endoscopique selon l'une quelconque des revendications précédentes, dans lequel l'élément de réglage (30) présente un élément de support (42) de forme au moins approximativement circulaire, sur lequel sont disposés le premier dispositif de butée (62) et le deuxième dispositif de butée (64) au moins approximativement dans la direction périphérique de l'élément de support (42).

10. Instrument endoscopique selon l'une quelconque des revendications précédentes, comprenant en outre un dispositif de commande qui est réalisé de manière à détecter une valeur de mesure d'une flexion de la première portion (50) et à commander l'actionneur (32) en tenant compte de la valeur de mesure.

11. Instrument endoscopique selon l'une quelconque des revendications précédentes, dans lequel un troisième axe de rotation (86) de l'élément de commande (14) et un quatrième axe de rotation (88) de l'élément intermédiaire (40) coïncident.

12. Instrument endoscopique selon l'une quelconque des revendications précédentes, dans lequel l'élément de mesure (56) présente au moins une jauge extensométrique.

13. Instrument endoscopique selon l'une quelconque des revendications précédentes, dans lequel la première portion (50) et la deuxième portion (52) sont réalisées au moins approximativement en forme de poutre, convergent vers un point commun (82) et sont orientées suivant un angle (a) par rapport à ce point commun (82).
